(19) Europäisches Patentamt
European Patent Office
Office européen des brevets

(11) **EP 4 174 473 A1**

(12) **EUROPEAN PATENT APPLICATION**

(43) Date of publication:
**03.05.2023 Bulletin 2023/18**

(51) International Patent Classification (IPC):
**G01N 21/31** *(2006.01)* **G01N 21/64** *(2006.01)*
**G01N 33/493** *(2006.01)*

(21) Application number: **21306538.6**

(22) Date of filing: **02.11.2021**

(52) Cooperative Patent Classification (CPC):
**G01N 21/31; G01N 21/6486; G01N 33/493;**
G01N 2201/1211; G01N 2201/129

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Usense**
**91300 Masy (FR)**

(72) Inventor: **OUERIEMMI, Amir**
**91300 Massy (FR)**

(74) Representative: **Icosa**
**83 avenue Denfert-Rochereau**
**75014 Paris (FR)**

(54) **METHOD AND SYSTEM FOR MEASURING URINE BIOMARKERS**

(57) The invention relates to a computer-implemented method for measuring a value of at biomarker of a urine sample, the method including the steps:
- based on a raw optical spectrum of the urine sample acquired over first wavelengths, determining a corresponding high-resolution optical spectrum of the urine sample, the high-resolution optical spectrum being defined over a plurality of second wavelengths, a wavelength interval between two successive second wavelengths being less than a wavelength interval between two successive first wavelengths; and
- implementing a biomarker model to estimate a value of the biomarker based at least on the determined high-resolution optical spectrum, the biomarker model being trained based on a training dataset including input data comprising a plurality of reference optical spectra, and output data comprising a plurality of reference values of the biomarker, each reference value of the biomarker being associated to a respective reference optical spectrum.

**FIG. 1**

## Description

### FIELD OF INVENTION

The invention concerns a computer-implemented method for measuring a value of at least one biomarker of a urine sample.

The invention applies to the field of urinalysis, and more precisely to the measurement of biomarkers concentration.

### BACKGROUND OF INVENTION

Clinical urine tests, also known as urinalysis, are an examination of urine for certain biomarkers. Urinalysis is widely used for health check and/or diagnosis of diseases.

Examination of physical properties of urine is recommended as an initial evaluation of urine samples before more sophisticated tests (chemical, immunologic) are performed in laboratories. Currently, these urine physical properties examinations are mostly performed using different machines that measure different parameters or biomarker values, for instance based on an optical spectrum of the urine sample. More specifically, a high-resolution spectrum of a urine sample needs to be acquired using a dedicated laboratory equipment in order to derive the biomarker value from said high-resolution spectrum.

However, such method is not fully satisfactory.

Indeed, such method requires expensive pieces of equipment to measure different parameters, such as high-resolution optical spectra of the urine (with a resolution of typically 0.5 nm) and require human expertise for interpretation of said spectra. Such method is also time consuming, since the results must be aggregated with results of other chemical and/or physical analysis in a report and returned to the practitioner. Thus, the final results are generated long after the sampling, causing a delayed diagnosis and, consequently, unwanted and unnecessary stress to the patient.

Therefore, a purpose of the invention is to provide a method for measuring values of biomarkers of a urine sample that is more cost-effective and less time-consuming than the known methods.

### SUMMARY

To this end, the present invention is a method of the aforementioned type, including, for each biomarker, the steps:

- based on a raw optical spectrum of the urine sample acquired over a plurality of first wavelengths of a predetermined wavelength range, determining a corresponding high-resolution optical spectrum of the urine sample, the high-resolution optical spectrum being defined over a plurality of second wavelengths of the predetermined wavelength range, a wavelength interval between two successive second wavelengths being less than a wavelength interval between two successive first wavelengths; and

- implementing at least one corresponding biomarker model to estimate a value of the biomarker based at least on the determined high-resolution optical spectrum, each biomarker model being trained based on a training dataset including input data comprising a plurality of reference optical spectra acquired over at least part of the predetermined wavelength interval, and output data comprising a plurality of reference values of the biomarker, each reference value of the biomarker being associated to a respective reference optical spectrum.

Indeed, thanks to the invention, a precise determination of each biomarker value can be achieved without resorting to expensive equipment to measure multiple high-resolution optical spectra of the urine sample, then aggregating said multiple spectra in order to obtain a final optical spectrum on a broad wavelength range, which will serve as a basis for determining biomarker values. On the contrary, a relatively cheap device for point of care urinalysis, which has lower resolution (around 30 nm) but wider wavelength range (typically infrared, visible and ultraviolet), can be used: in this case, said device almost instantaneously provides a raw optical spectrum. The high-resolution optical spectrum associated to each biomarker is then quickly determined, based on the raw optical spectrum, over said broad wavelength range. Moreover, using biomarker models that are specific to each biomarker improves the relevancy of the determined high-resolution optical spectrum. As a result, quick and reliable measurements of biomarker values are achieved.

According to other advantageous aspects of the invention, the method includes one or more of the following features, taken alone or in any possible combination:

- for each biomarker model, the input data of the corresponding training dataset also include a plurality of reference conductivity measurement, each reference value of the biomarker being further associated to a respective reference conductivity measurement, and for each biomarker, the method further comprising implementing each corresponding biomarker model to estimate a value of said biomarker based on the corresponding high-resolution optical spectrum and on an acquired conductivity measurement of the urine sample;

- the method further comprises correcting the acquired raw optical spectrum based on a temperature of the urine sample;

- the acquired conductivity measurement includes at least one conductivity value acquired between 0 Hz and 1 MHz;

- the method further comprises correcting the acquired conductivity measurement based on a temperature of the urine sample;

- determining each high-resolution optical spectrum comprises performing a spectral reconstruction algorithm on the corresponding raw optical spectrum based on a transmission curve of each input optics of an optical spectrometer used to acquire said raw optical spectrum;

- each biomarker is associated to a corresponding relevant wavelength range, and for each raw optical spectrum, determining the corresponding high-resolution optical spectrum includes identifying a base high-resolution optical spectrum, among a plurality of stored base high-resolution optical spectra, that is the closest to said raw optical spectrum over the relevant wavelength range with regard to a predetermined metric, the identified base high-resolution optical spectrum forming the high-resolution optical spectrum;

- for each raw optical spectrum, determining the corresponding high-resolution optical spectrum includes identifying a base high-resolution optical spectrum, among a plurality of stored base high-resolution optical spectra, that is the closest to said raw optical spectrum over the predetermined wavelength range with regard to a predetermined metric, the identified base high-resolution optical spectrum forming the high-resolution optical spectrum;

- the predetermined wavelength range overlaps with at least one of the following wavelength sub-ranges: [270 nm; 400 nm], [390 nm; 1100 nm] and/or [800 nm; 2600 nm];

- the method further comprises:

  - step of determining whether the estimated values of at least two biomarkers satisfy a predetermined physiological relationship; and
  - generating a warning signal if not;

- the method further comprises a step of data clustering, based on at least one raw optical spectrum, to assign the urine sample to one of a plurality of predetermined patient classes, at least one predetermined patient class being associated to a corresponding pathology or health status, the biomarker model depending on the assigned patient class;

- each biomarker is associated to at least two distinct biomarker models, and, for each biomarker, the corresponding estimated value is a function of a result provided by each biomarker model;

- the method further comprises a step of prescreening including:

  - performing data analysis on each raw optical spectrum to determine, based on features of the raw optical spectrum, whether the corresponding urine sample is associated to a patient having a predetermined atypical status;
  - generating an alert signal if the urine sample is found to be associated to a patient having a predetermined atypical status.

The invention also relates to a computer program product comprising instructions which, when the program is executed by a computer, cause the computer to carry out the steps of the method as defined previously.
The invention also relates to a system for measuring a value of at least one biomarker of a urine sample, the system comprising a processing unit configured to, for each biomarker:

- determine, based on a raw optical spectrum of the urine sample acquired over a plurality of first wavelengths of a predetermined wavelength range, a corresponding high-resolution optical spectrum of the urine sample, the high-resolution optical spectrum being defined over a plurality of second wavelengths of the predetermined wavelength range, a wavelength interval between two successive second wavelengths being less than a wavelength interval between two successive first wavelengths; and
- implement at least one corresponding biomarker model to estimate a value of the biomarker based at least on the determined high-resolution optical spectrum, each biomarker model being trained based on a training dataset including input data comprising a plurality of reference optical spectra acquired over at least part of the predetermined wavelength interval, and output data comprising a plurality of reference values of the biomarker, each reference value of the biomarker being associated to a respective reference optical spectrum.

## BRIEF DESCRIPTION OF THE DRAWINGS

**Figure 1** is a schematical representation of a urinalysis system according to the invention;

**Figure 2** is a graph showing a raw optical spectrum and a corresponding high-resolution optical spectrum determined by the urinalysis system of figure 1; and

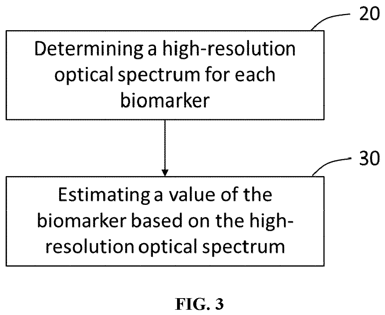

**Figure 3** is a chart showing a urinalysis method according to the invention.

## DETAILED DESCRIPTION

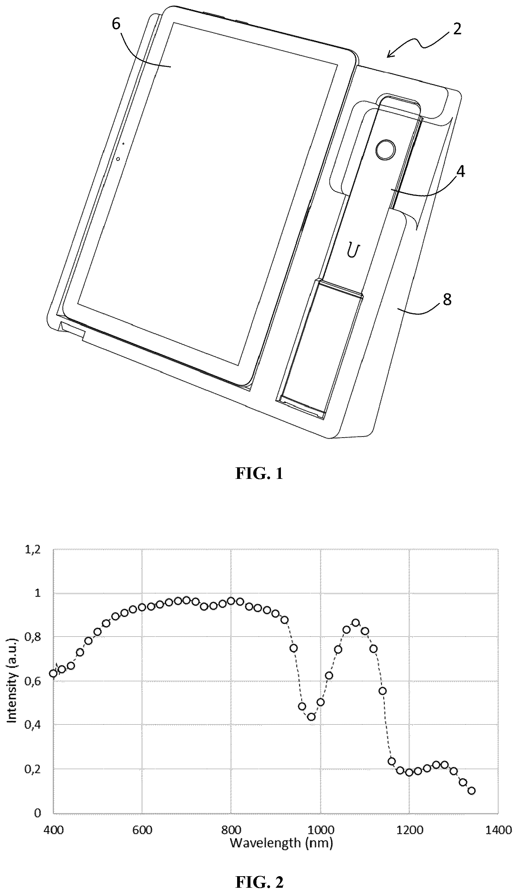

A urinalysis system 2 according to the invention is shown on figure 1.

The urinalysis system 2 is configured to acquire data relating to a urine sample of a patient, and to measure a value of at least one biomarker of said urine sample.

More precisely, the urinalysis system 2 comprises at least one probe 4 and a processing unit 6. Optionally, the urinalysis system 2 includes a docking station 8 for allowing communication between the probe 4 and the processing unit 6. Alternatively, the processing unit 6 and each probe 4 are configured to directly communicate with each other.

### *Probe 4*

The probe 4 is configured to acquire at least one raw optical spectrum of the urine sample, over a plurality of first wavelengths of a predetermined wavelength range. In other words, the raw optical spectrum comprises a plurality of couples, each couple comprising a first wavelength and a corresponding measured light intensity. Said intensity may be representative of a transmission of the urine sample at the corresponding first wavelength, or a fluorescence of the urine sample at the corresponding first wavelength. An example of such raw optical spectrum is shown on figure 2, and corresponds to the circles on the graph. In this example, a first wavelength interval between two successive first wavelengths is 30 nm.

To acquire said raw optical spectrum, the probe 4 includes an optical spectrometer (not shown), such as a miniaturized optical spectrometer. Preferably, for each first wavelength, the optical spectrometer includes a corresponding light detection member, which preferably includes a photodetector. Preferably, each photodetector is provided, at an optical input thereof, with input optics. For instance, said input optics comprise an optical filter, said optical filter having a known transmission curve. Alternatively, or in addition, the input optics comprise an interferometer, such as a Fabry-Perot interferometer, preferably having an adjustable transmission curve.

Preferably, the predetermined wavelength range overlaps with at least one of the following wavelength sub-ranges: [270 nm; 400 nm], [390 nm; 1100 nm] and/or [800 nm; 2600 nm]. In other words, the predetermined wavelength range is at least partially included in the [270 nm; 2600 nm] interval. This is advantageous, because the main urine components can be identified in these wavelength ranges. Moreover, is such wavelength range, the cost of the optical and electronic components is generally very low compared to other wavelength ranges: the cost of the probe 4.

Preferably, the probe 4 is further configured to acquire a conductivity measurement of the urine sample.

Said conductivity measurement includes at least one conductivity value, preferably acquired at 0 Hz (*i.e.,* a conductivity value associated to a DC current), and/or at frequencies higher than 0 Hz (*i.e.,* a conductivity value associated to an AC current), for instance up to 1 MHz. For instance, the conductivity measurement is a conductivity spectrum acquired in a frequency range included in the [0 Hz; 1 MHz] range.

Preferably, the probe 4 is also configured to acquire a temperature value of the urine sample.

The probe 4 is, for instance, similar to the probe disclosed in European patent application EP 20 306 576.

### *Processing unit 6*

The processing unit 6 will now be disclosed.

According to the invention, the expression "processing unit" should not be construed to be restricted to hardware capable of executing software, and refers in a general way to a processing device, which can, for example, include a microprocessor, an integrated circuit, or a programmable logic device (PLD). The processing unit 6 may also encompass one or more Graphics Processing Units (GPU) or Tensor Processing Units (TSU), whether exploited for computer graphics and image processing or other functions. Additionally, the instructions and/or data enabling to perform associated and/or resulting functionalities may be stored on any processor-readable medium such as, e.g., an integrated circuit, a hard disk, a CD (Compact Disc), an optical disc such as a DVD (Digital Versatile Disc), a RAM (Random-Access Memory) or a ROM (Read-Only Memory). Instructions may be notably stored in hardware, software, firmware or in any combination thereof.

### *Biomarkers*

The processing unit 6 is configured to measure a value of at least one biomarker of the urine sample.

The aforementioned biomarker may be a concentration or a concentration range of a predetermined chemical compound in the urine sample, such as a mineral, for instance at least one of: sodium, potassium, chloride, phosphorus, magnesium, and calcium.

Alternatively, or in addition, the biomarker may also be a biomarker representative of a fluid balance of the patient, for instance at least one of: urine specific gravity and osmolality.

Alternatively, or in addition, the biomarker may also be a biomarker representative of patient kidney function. For instance, such biomarker is at least one of: a concentration or a concentration range of creatinine, an indication relating to the presence or the absence of albumin in the urine sample, a concentration or a concentration range of albumin, and a concentration or concentration range of ammonia. The biomarker may also be an indication relating to the presence or the absence of proteins in the urine sample, or even a concentration or a concentration range of total protein in the urine sample.

Alternatively, or in addition, the biomarker may also be a biomarker indicative of the presence of kidney stone(s) in one or both kidney(s) of the patient. For instance, such biomarker is at least one of a concentration or a concentration range of citrate, a concentration or a concentration range of oxalate and an indication relating to the presence of crystals in the urine sample.

Alternatively, or in addition, the biomarker may also be a biomarker representative of metabolism of the patient. For instance, such biomarker may be at least one of: a pH or a pH range of the urine sample, a concentration or a concentration range of uric acid, an indication relating to the presence or the absence of ketones, a concentration or a concentration range of ketones, a concentration or a concentration range of bilirubin, a concentration or a concentration range of urobilinogen and a concentration, and a concentration range of urea.

Alternatively, or in addition, the biomarker may also be a biomarker indicative of hyperglycemia in the patient. For instance, such biomarker may be an indication relating to presence or the absence of glucose, or a concentration range or a concentration of glucose.

Alternatively, or in addition, the biomarker may also be a biomarker indicative of an infection of the patient. For instance, such biomarker may relate to at least one of: hematuria (*i.e.,* an indication relating to the presence or the absence thereof, or a concentration range or a concentration thereof), leukocytes (*i.e.,* an indication relating to the presence or absence, or a concentration range or concentration thereof), and bacteria (*i.e.,* an indication relating to the presence or the absence, range of concentration or concentration thereof).

Alternatively, or in addition, the biomarker may also be a biomarker indicative of a specific pathology such as, for example, rare disease. For instance, such biomarker may be at least one of: an indication relating to the presence or the absence of porphyria, a concentration or a concentration range of porphyria, a concentration or a concentration range of oxalate (indicating hyperoxaluria), and an indication relating to the presence or the absence of endogenous fluorophores.

Alternatively, or in addition, the biomarker may also be a biomarker indicative of patient oxidative stress.

Alternatively, or in addition, the biomarker may also be a biomarker indicative of patient immunity status.

Alternatively, or in addition, the biomarker may also be a biomarker indicative of patient diet quality.

*Configuration of the processing unit 6*

More specifically, the processing unit 6 is configured to determine the value of each biomarker based at least on a raw optical spectrum of the urine sample. Said raw optical spectrum is, for instance, received from the probe 4.

The processing unit 6 is configured to perform a measurement method, a workflow of which is schematically shown on figure 2. Said measurement method comprises a high-resolution optical spectrum determination step 20 (hereinafter "determination step 20") and a biomarker value estimation step 30 (hereinafter "estimation step 30"). More precisely, the processing unit 6 is configured to determine, during the determination step 20, and for each biomarker, a corresponding high-resolution optical spectrum based on the raw optical spectrum. Moreover, for each biomarker, the processing unit 6 is configured to estimate a respective value based at least on the determined high-resolution optical spectrum.

A detailed description of steps 20 and 30 will now be provided.

*Determination step 20*

As mentioned previously, the processing unit 6 is configured to determine, during the determination step 20, and for each biomarker, a corresponding high-resolution optical spectrum based on the raw optical spectrum.

Such high-resolution optical spectrum is defined over a plurality of second wavelengths of the predetermined wavelength range. Moreover, a second wavelength interval between two successive second wavelengths is less than a first wavelength interval between two successive first wavelengths. In other words, the high-resolution optical spectrum has a spectral resolution that is greater than a spectral resolution of the raw optical spectrum, while being similar in shape to the raw optical spectrum. An example of such high-resolution optical spectrum is shown on figure 2, and corresponds to the dashed line on the graph. In this example, a second wavelength interval between two successive first wavelengths is 0.5 nm.

In other words, the high-resolution optical spectrum is the spectrum having a higher resolution than the raw optical and that most closely matches the data couples of the raw optical spectrum.

Preferably, in order to determine the high-resolution optical spectrum associated to a given biomarker, the processing unit 6 is configured to perform spectral reconstruction on the corresponding raw optical spectrum based on a predetermined reconstruction algorithm. Said spectral reconstruction algorithm is based on a transmission curve of the input optics of each optical spectrometer used to acquire said raw optical spectrum (generally the optical spectrometer included in the probe 4).

For instance, the processing unit is configured to solve M equations of the following form:

$$b_i = \int f(\lambda)\,\phi_i(\lambda)h(\lambda)d\lambda + e_i$$

where $b_i$ is an amplitude of a detection signal output by light detection member i, "i" being an integer comprised between 1 and M, M being an integer lower than or equal to N, N being the total number of points of the raw optical spectrum, that is the total number of light detection members;

$f(\lambda)$ is the light spectrum reaching each light detection member (*i.e.,* the high-resolution optical spectrum);

$\phi_i(\lambda)$ is the transmission curve of the input optics of light detection member i;

$h(\lambda)$ is a response function of the photodetector of light detection member i; and $e_i$ is a measurement error.

Alternatively, at least one biomarker is associated to a corresponding relevant wavelength range. In this case, starting from the raw optical spectrum, and in order to determine the high-resolution optical spectrum associated to a given biomarker, the processing unit 6 is configured to identify, among a plurality of stored base high-resolution optical spectra, the base high-resolution optical spectrum that is the closest to said raw optical spectrum over the relevant wavelength range with regard to a predetermined metric. In this case, the identified base high-resolution optical spectrum forms the high-resolution optical spectrum.

As an example, the processing unit 6 is configured to implement a closest distance matching algorithm (such as a k-nearest neighbors algorithm) in order to determine the high-resolution optical spectrum, based on the raw optical spectrum and each base high-resolution optical spectrum.

Advantageously, the processing unit 6 is configured to correct, prior to the determination of the high-resolution optical spectrum, the acquired raw optical spectrum based on a temperature of the urine sample. This allows to compensate for any potential temperature-related bias of the optical spectrometer of the probe 4, notably temperature-induced spectral variations such as the position and intensity of spectral absorption bands of the chemical compounds in the urine sample.

*Estimation step 30*

Moreover, for each biomarker, the processing unit 6 is configured to estimate, during the estimation step 30, a respective value based at least on the determined high-resolution optical spectrum.

More precisely, in order to estimate a value of a given biomarker, the processing unit 6 is configured to implement at least one corresponding biomarker model to estimate said value based at least on the high-resolution optical spectrum.

Each biomarker model has been trained prior to the implementation of the estimation step 30. More precisely, for each biomarker, each corresponding biomarker model has been trained based on a training dataset including input data comprising a plurality of reference optical spectra acquired over at least part of the predetermined wavelength interval, and output data comprising a plurality of reference values of the biomarker, each reference value of the biomarker being associated to a respective reference optical spectrum. Preferably, the reference optical spectra have the same resolution as the high-resolution optical spectra.

Advantageously, for at least one biomarker, the processing unit 6 is further configured to determine the corresponding value based on the conductivity measurement of the urine sample. In this case, for at least one biomarker model corresponding to a given biomarker, the input data of the corresponding training dataset also include a plurality of reference conductivity measurements. As a result, each reference value of the biomarker is further associated to a respective reference conductivity measurement. In this case, for each concerned biomarker, the processing unit 6 is further configured to implement each corresponding biomarker model to estimate a value of said biomarker based on the corresponding high-resolution optical spectrum and on the acquired conductivity measurement of the urine sample. This is advantageous, as the inventors have realized that a conductivity measurement leads to a more precise estimation of the value of almost each of the aforementioned biomarker.

In this case, the processing unit 6 is advantageously also configured to correct the acquired conductivity measurement based on a temperature of the urine sample prior to the estimation of each biomarker. This is advantageous, as such step allows to compensate for the temperature-dependency of the conductivity of a solution.

Advantageously, each biomarker is associated to at least two distinct biomarker models. In order to be distinct from each other, said biomarker models have been trained based on different training datasets and/or are of different types (*e.g.,* an artificial neural network, a decision tree, a support-vector machine, a Bayesian network and so on). In this case, for each biomarker, the corresponding estimated value is a function of a result provided by each of the associated biomarker models. For instance, said estimated value is equal to the mean value of the results provided by all the biomarker models. According to another example, the number of biomarker models is greater than or equal to 3, and said estimated value is equal to the value which has the highest number of occurrences. Preferably, said estimated value is equal to the mean value of the results provided by all the biomarker models if a relative difference between the results provided by the biomarker models is within a predetermined tolerance range. In this case, if said mean value is outside the tolerance range, the processing unit 6 may be configured to output an alert signal.

Advantageously, for at least two biomarkers, the processing unit 6 is configured to determine, after the values of said biomarkers have been estimated, whether the corresponding estimated values satisfy a predetermined physiological relationship. In this case, the processing unit 6 is further configured to generate a warning signal if the predetermined physiological relationship is not satisfied. This is advantageous, as such step allows to check for consistency between the estimated values of the biomarkers, and/or allow detection of abnormalities (such as health issues) on the patient side.

Advantageously, the processing unit 6 is configured to perform, prior to the implementation of each biomarker models, a step of data clustering, based on at least one raw optical spectrum, to assign the urine sample to one of a plurality of predetermined patient classes, at least one predetermined patient class being associated to a corresponding pathology or health status. In this case, at least one biomarker model depends on the assigned patient class. For instance, for a given biomarker, the processing unit 6 is configured to select a biomarker model for estimating the value of said biomarker depending on the patient class to which the patient has been assigned. As a result, estimation of each biomarker value is more reliable. As an example, glycosuria can be encountered in patient suffering diabetes, requiring the use of specific algorithms to estimate glucose concentration.

For instance, such data clustering includes projecting the raw optical spectra in a predetermined clustering latent space. Such projection is, for example, based on the implementation of a trained artificial intelligence model. Alternatively, such projection includes performing a predetermined data transformation algorithm, such as principal component analysis. By comparing the result of such projection to predetermined patient classes, a patient class is assigned to the current patient. The assigned patient class is, for instance, the patient class that is the closest to the projected raw optical spectrum, with respect to a predetermined metric.

According to an advantageous embodiment, the processing unit 6 is also configured to perform a step of prescreening, before the determination step 20, that is to say based on each raw optical spectrum. Such step of prescreening is aimed at detecting a patient health status prior to, or without, computing each biomarker value. Such prescreening is based on the fact that, for a predetermined set (hereinafter referred to as "atypical status" or "atypical health status") of health statuses, the raw optical spectrum exhibits specific features. In other words, during prescreening, the processing unit 6 performs data analysis on each raw optical spectrum to determine, based on the features of the raw optical spectrum, whether the urine sample is associated to a patient having a predetermined atypical status.

For instance, the atypical status may be one of: metabolic malfunction, drug use, doping (*i.e.,* use of performance enhancing drugs), supplement intake, absorption of one or more heavy metals, absorption of one or more drugs belonging to predetermined classes (such as: analgesics, antibiotics, anticoagulants, antidepressants, anticarcinogens, anticonvulsants, neuroleptics, antiviral drugs, sedatives, stimulants, diabetes medications, or cardiovascular drugs).

Preferably, the step of prescreening includes projecting each raw optical spectrum on a prescreening latent space (*e.g.,* by performing dimensionality reduction techniques, like principal component analysis or embedding methods). In this case, the processing unit 6 is configured to determine that the raw optical spectrum is associated to a patient having an atypical status if the projected raw optical spectrum belongs to a predetermined region of the prescreening latent space, associated to said atypical status.

Alternatively, or in addition, the step of prescreening comprises determining whether, for a predetermined set of wavelengths, the amplitude of the raw optical spectrum is higher than a respective threshold. In this case, the processing unit 6 is configured to determine that the raw optical spectrum is associated to a patient having an atypical status if, for at least part of the predetermined set of wavelengths, the amplitude of the raw optical spectrum is higher that the respective threshold.

This latter aspect of the invention results from the fact that, due to the presence of exogenous chemical components in the urine sample, a plurality of fluorescence peaks is observed in the raw optical spectrum, for instance, in the [500 nm; 1100 nm] wavelength range. This is, for instance, the case if the patient suffers from porphyria.

Preferably, the processing unit 6 is also configured to so that it does not perform steps 20, 30 if the patient associated to the current urine sample is found to have an atypical status.

The processing unit 6 is, preferably, further configured to generate an alert signal if the patient associated to the current urine sample is found to have an atypical status.

Operation of the urinalysis system 2 will now be described.

The processing unit 6 receives a raw optical spectrum of the urine sample of a patient. As mentioned previously, the raw optical spectrum includes a plurality of couples, each couple comprising a first wavelength and a corresponding measured light intensity.

Advantageously, the processing unit 6 corrects the acquired raw optical spectrum based on a temperature of the urine sample.

Then, during an optional step of prescreening, the processing unit 6 performs data analysis on each raw optical spectrum to determine, based on the features of the raw optical spectrum, whether the urine sample is associated to a patient having a predetermined atypical status. If the patient has an atypical status, the processing unit 6 preferably generates an alert signal and does not perform further processing of the raw optical spectrum.

Then, during the determination step 20, and for each biomarker, the processing unit 6 determines a corresponding high-resolution optical spectrum based on the raw optical spectrum. Each high-resolution optical spectrum includes a plurality of couples, each couple comprising a second wavelength and a corresponding computed light intensity. A second wavelength interval between two successive second wavelengths is smaller, for instance at least ten times smaller, than a first wavelength interval between two successive first wavelengths of the raw optical spectrum.

Preferably, in order to determine the high-resolution optical spectrum associated to a given biomarker, the processing unit 6 performs a spectral reconstruction algorithm on the corresponding raw optical spectrum.

Advantageously, the processing unit 6 also performs a step of data clustering, based on at least one raw optical spectrum, to assign the urine sample to one of a plurality of predetermined patient classes.

Then, during the estimation step 30, for each biomarker, the processing unit 6 estimates a respective value based at least on the determined high-resolution optical spectrum. More precisely, to estimate a value of a given biomarker, the processing unit 6 implements at least one corresponding biomarker model to estimate said value. Preferably, each biomarker model is selected based on the assigned patient class.

Advantageously, for at least one biomarker, the processing unit 6 is further configured to determine the corresponding value based on a conductivity measurement of the urine sample (which has advantageously also been corrected based on a temperature of the urine sample).

As a result, the value of each biomarker is measured.

## Claims

1. A computer-implemented method for measuring a value of at least one biomarker of a urine sample, the method including, for each biomarker, the steps:

   - based on a raw optical spectrum of the urine sample acquired over a plurality of first wavelengths of a predetermined wavelength range, determining a corresponding high-resolution optical spectrum of the urine sample, the high-resolution optical spectrum being defined over a plurality of second wavelengths of the predetermined wavelength range, a wavelength interval between two successive second wavelengths being less than a wavelength interval between two successive first wavelengths; and
   - implementing at least one corresponding biomarker model to estimate a value of the biomarker based at least on the determined high-resolution optical spectrum, each biomarker model being trained based on a training dataset including input data comprising a plurality of reference optical spectra acquired over at least part of the predetermined wavelength interval, and output data comprising a plurality of reference values of the biomarker, each reference value of the biomarker being associated to a respective reference optical spectrum.

2. The method according to claim **1,** wherein, for each biomarker model, the input data of the corresponding training dataset also include a plurality of reference conductivity measurement, each reference value of the biomarker being further associated to a respective reference conductivity measurement, and for each biomarker, the method further comprising implementing each corresponding biomarker model to estimate a value of said biomarker based on the corresponding high-resolution optical spectrum and on an acquired conductivity measurement of the urine sample.

3. The method according to claim **1** or **2,** further comprising correcting the acquired raw optical spectrum based on a temperature of the urine sample.

4. The method according to any one of claims **1** to **3,** wherein the acquired conductivity measurement includes at least one conductivity value acquired between 0 Hz and 1 MHz.

5. The method according to any one of claims **4,** further comprising correcting the acquired conductivity measurement based on a temperature of the urine sample.

6. The method according to any one of claims **1** to **5,** wherein determining each high-resolution optical spectrum comprises performing a spectral reconstruction algorithm on the corresponding raw optical spectrum based on a transmission curve of each input optics of an optical spectrometer used to acquire said raw optical spectrum.

7. The method according to any one of claims **1** to **6,** wherein each biomarker is associated to a corresponding relevant wavelength range, and wherein, for each raw optical spectrum, determining the corresponding high-resolution optical spectrum includes identifying a base high-resolution optical spectrum, among a plurality of stored base high-resolution optical spectra, that is the closest to said raw optical spectrum over the relevant wavelength range with regard to a predetermined metric, the identified base high-resolution optical spectrum forming the high-resolution optical spectrum.

8. The method according to any one of claims **1** to **7,** wherein, for each raw optical spectrum, determining the corresponding high-resolution optical spectrum includes identifying a base high-resolution optical spectrum, among a plurality of stored base high-resolution optical spectra, that is the closest to said raw optical spectrum over the predetermined wavelength range with regard to a predetermined metric, the identified base high-resolution optical spectrum forming the high-resolution optical spectrum.

9. The method according to any one of claims **1** to **8,** wherein the predetermined wavelength range overlaps with at least one of the following wavelength sub-ranges: [270 nm; 400 nm], [390 nm; 1100 nm] and/or [800 nm; 2600 nm].

10. The method according to any one of claims **1** to **9,** further comprising:

    - a step of determining whether the estimated values of at least two biomarkers satisfy a predetermined physiological relationship; and
    - generating a warning signal if not.

11. The method according to any one of claims **1** to **10,** further comprising a step of data clustering, based on at least one raw optical spectrum, to assign the urine sample to one of a plurality of predetermined patient classes, at least one predetermined patient class being associated to a corresponding pathology or health status, the biomarker model depending on the assigned patient class.

12. The method according to any one of claims **1** to **11,** wherein each biomarker is associated to at least two distinct biomarker models, and, for each biomarker, the corresponding estimated value is a function of a result provided by each biomarker model.

13. The method according to any one of claims **1** to **12,** further comprising a step of prescreening including:

    - performing data analysis on each raw optical spectrum to determine, based on features of the raw optical spectrum, whether the corresponding urine sample is associated to a patient having a predetermined atypical status;
    - generating an alert signal if the urine sample is found to be associated to a patient having a predetermined atypical status.

14. Computer program product comprising instructions which, when the program is executed by a computer, cause the computer to carry out the steps of the method according to any one of claims **1** to **13.**

15. System (2) for measuring a value of at least one biomarker of a urine sample, the system (2) comprising a processing unit (6) configured to, for each biomarker:

    - determine, based on a raw optical spectrum of the urine sample acquired over a plurality of first wavelengths of a predetermined wavelength range, a corresponding high-resolution optical spectrum of the urine sample, the high-resolution optical spectrum being defined over a plurality of second wavelengths of the predetermined wavelength range, a wavelength interval between two successive second wavelengths being less than a wavelength interval between two successive first wavelengths; and
- implement at least one corresponding biomarker model to estimate a value of the biomarker based at least on the determined high-resolution optical spectrum, each biomarker model being trained based on a training dataset including input data comprising a plurality of reference optical spectra acquired over at least part of the predetermined wavelength interval, and output data comprising a plurality of reference values of the biomarker, each reference value of the biomarker being associated to a respective reference optical spectrum.

**FIG. 1**

**FIG. 2**

Determining a high-resolution
optical spectrum for each
biomarker — 20

Estimating a value of the
biomarker based on the high-
resolution optical spectrum — 30

**FIG. 3**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/323949 A1 (CARVALHO SOUSA NUNO JORGE [PT] ET AL) 24 October 2019 (2019-10-24) | 1,4,6-9, 11,12, 14,15 | INV. G01N21/31 G01N21/64 G01N33/493 |
| Y | * paragraphs [0004] – [0011], [0033] – [0034], [0039] * <br> * paragraphs [0052], [0059] – [0060] * <br> * paragraphs [0079], [0088], [0093] * <br> * paragraphs [0136], [0169] – [0177]; figures 4A-4C; tables 1-5 * <br> ----- | 2,3,5, 10,13 | |
| Y | US 2021/311018 A1 (HARRINGTON J BRIAN [US] ET AL) 7 October 2021 (2021-10-07) <br> * paragraphs [0002], [0003], [0010], [0015] – [0016]; figure 2 * <br> * paragraphs [0018], [0021] – [0028], [0032], [0044]; figures 3A,3B,4A * <br> ----- | 3,5 | |
| Y | US 5 580 794 A (ALLEN MICHAEL P [US]) 3 December 1996 (1996-12-03) <br> * column 1, line 8 – line 15 * <br> * column 7, line 41 – column 10, line 56; figure 2 * <br> * example 6 * <br> ----- | 2 | TECHNICAL FIELDS SEARCHED (IPC) <br><br> G01N |
| Y | WO 2016/154262 A1 (WELLMETRIS LLC [US]) 29 September 2016 (2016-09-29) <br> * paragraphs [0005] – [0008]; figure 1 * <br> * claims 24-27 * <br> ----- | 10,13 | |

The present search report has been drawn up for all claims

| Place of search | Date of completion of the search | Examiner |
|---|---|---|
| Munich | 21 April 2022 | Consalvo, Daniela |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

.......................................................................

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 21 30 6538

21-04-2022

| Patent document cited in search report | | Publication date | Patent family member(s) | | Publication date |
|---|---|---|---|---|---|
| US 2019323949 | A1 | 24-10-2019 | US | 2015369725 A1 | 24-12-2015 |
| | | | US | 2019323949 A1 | 24-10-2019 |
| | | | WO | 2014118601 A1 | 07-08-2014 |
| | | | WO | 2014118745 A1 | 07-08-2014 |
| US 2021311018 | A1 | 07-10-2021 | US | 2021311018 A1 | 07-10-2021 |
| | | | WO | 2020131349 A1 | 25-06-2020 |
| US 5580794 | A | 03-12-1996 | AU | 7563294 A | 21-03-1995 |
| | | | CA | 2170402 A1 | 02-03-1995 |
| | | | EP | 0722563 A1 | 24-07-1996 |
| | | | JP | 3035352 B2 | 24-04-2000 |
| | | | JP | H09503581 A | 08-04-1997 |
| | | | US | 5580794 A | 03-12-1996 |
| | | | WO | 9506240 A1 | 02-03-1995 |
| WO 2016154262 | A1 | 29-09-2016 | CA | 2979864 A1 | 29-09-2016 |
| | | | EP | 3274706 A1 | 31-01-2018 |
| | | | HK | 1250391 A1 | 14-12-2018 |
| | | | JP | 2018512587 A | 17-05-2018 |
| | | | US | 2018049723 A1 | 22-02-2018 |
| | | | WO | 2016154262 A1 | 29-09-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 20306576 A